# EUROPEAN PATENT APPLICATION

(11) **EP 2 650 023 A1**
(43) Date of publication of application: **16.10.2013**
(21) Application number: 11847342.0
(22) Date of filing: 01.12.2011
(51) Int. Cl.: A61L 2/20, B65B 55/10

(54) **DISINFECTANT SOLUTION GASIFICATION DEVICE**

(30) Priority: 08.12.2010 JP 2010273639
(71) Applicant: Shikoku Kakoki Co., Ltd., Itano-gun Tokushima 771-0287 (JP)
(72) Inventor: ITOH, Yasumasa, Itano-gun Tokushima 771-0287 (JP); NISHIO, Yoji, Itano-gun Tokushima 771-0287 (JP); NISHIUCHI, Kazumasa, Itano-gun Tokushima 771-0287 (JP)
(74) Representative: Harding, Charles Thomas
(86) International application number: PCT/JP2011/006733
(87) International publication number: WO 2012/077307

(57) **Abstract**

Provided is a sterilizing liquid gasifying device which is excellent in sterilizing liquid gasifying efficiency, consumes a small amount of electric energy, is compact in shape, and enables the easy maintenance thereof. The sterilizing liquid gasifying device includes: a hot air duct 3 having a hot air inlet at one end and a hot air outlet at the other end; a hot air source 2 which supplies hot air having a temperature capable of gasifying a sterilizing liquid to the hot air inlet; a spray nozzle 4 which sprays a sterilizing liquid in the direction opposite to the flow of hot air ejected from the hot air outlet so as to make a sprayed and atomized sterilizing liquid collide with hot air; and a hermetically sealed gasifying tank 1 which surrounds at least the hot air outlet of the hot air duct and a spray portion at a distal end of the spray nozzle, wherein a gas discharge opening is formed on the gasifying tank on a side opposite to the spray portion at a distal end of the spray nozzle across the hot air outlet.

## Description

### Technical Field

The present invention relates to, for example, a gasifying device for gasifying sterilizing liquid used for sterilizing container packaging materials.

### Background Art

Conventionally, in filling liquid food such as milk or soft drink in a paper container or a plastic container such as a PET bottle, for the purpose of prolonging a shelf life of a product, a container is sterilized using a sterilizing agent such as hydrogen peroxide before liquid food is filled in the container. As a method for sterilizing a container, there has been known a method where a container is immersed into a sterilizing agent reservoir or a method where a sterilizing agent in a gaseous form or in a liquid form is sprayed into a container. With respect to a case where hydrogen peroxide is gasified and a packaging material such as a container is pasteurized or sterilized using gasified hydrogen peroxide, there has been proposed a method where hydrogen peroxide is gasified in a high temperature atmosphere by bringing hydrogen peroxide in an atomized form into contact with a heating element using a spray nozzle or by bringing hydrogen peroxide in a droplet form into contact with a heating element using a dropping nozzle (see for example, patent document 1).

The method which brings a sterilizing liquid into contact with the heating element exhibits high gasifying efficiency. In using the method, however, there exists a possibility that the spray nozzle will be clogged by a foreign material or by the precipitation of a stabilizing agent contained in the sterilizing liquid. To obviate these drawbacks, the present inventors previously proposed a sterilizing liquid gasifying device comprising: a vertical hot air duct having an inlet at an upper end thereof and an outlet at a lower end thereof; a hot air source which supplies hot air having a temperature capable of gasifying a sterilizing liquid to the inlet; a spray nozzle which sprays the sterilizing liquid into the inside of the hot air duct; and a plate heater which is arranged below the outlet in a state where the plate heater faces the outlet in an opposed manner, wherein a lower portion including the outlet of the hot air duct and the plate heater are surrounded by a hermetically sealed gasifying tank, and a gas discharge opening is formed in an upper portion of the gasifying tank (see for example, patent document 2).

The present inventors previously proposed a sterilizing agent gasifying device for sterilizing container packaging materials, comprising a hot air duct having an inlet at one end and an outlet at the other end; a hot air source which supplies hot air having a temperature capable of gasifying a sterilizing liquid to the inlet; and a spray means which sprays the sterilizing liquid into the inside of the hot air duct; wherein the spray means comprises a spray nozzle having a gas-liquid mixing double pipe structure which has a spray opening thereof facing the inside of the hot air duct, which constitution enables to suppress the generation of bubbles, decrease a load on a hydrogen peroxide liquid feed pump, thus to stabilize a liquid feeding state, and to enhance gasification efficiency (see for example, patent document 3).

Further, the present inventors have also proposed a device for sterilization which can monitor a flow rate of a sterilizing agent in a liquid form, comprising: a tank which stores a sterilizing agent in a liquid form; a spray means which sprays the sterilizing agent transported from the tank onto a subject to be sterilized; a guide pipe which makes the tank and the spray means communicate with each other and guides the sterilizing agent in a liquid form in the tank to the spray means; a transport means which is provided upstream of the tank or in the guide pipe and transports the sterilizing agent in a liquid form to the spray means through the guide pipe from the tank; a throttle means which is provided to the guide pipe downstream of the transport means for maintaining a pressure in the guide pipe upstream of the throttle means at a predetermined pressure; and a pressure detection means which is provided between the transport means and the throttle means (see for example, patent document 4).

There has also been proposed a gasifying device which can supply a large amount of sterilizing gas, which sprays a sterilizing agent into hot air, comprising a plurality of temperature sensors installed downstream of a spray nozzle, and monitors gasifying efficiency based on the temperature difference between values detected by the individual temperature sensors (see for example, patent document 5). The gasifying device adopts a system which determines that gasification is insufficient when the difference between values detected by the temperature sensors arranged adjacent to each other is large, and increases an output of an electric heater so as to increase a temperature of hot air. In this system, however, the control system is complicated, and it is necessary to secure a space for installing the plurality of sensors so that the device becomes large-sized. Further, when a sterilizing agent and hot air are not mixed together uniformly, temperatures which the temperature sensors detect are not stabilized thus making the stable generation of a sterilizing gas difficult.

### Prior Art Documents

### Patent Documents

Patent Document 1: Japanese Unexamined Patent Application Publication No. 3-224469
Patent Document 2: Japanese Unexamined Patent Application Publication No. 2001-276189
Patent Document 3: Japanese Unexamined Patent Application Publication No. 2007-20744
Patent Document 4: Japanese Unexamined Patent Application Publication No. 2005-200027
Patent Document 5: Japanese Unexamined Patent Application Publication No. 2003-339829

### Summary of the Invention

### Object to be Solved by the Invention

To pasteurize or sterilize a packing material such as a container by using a gasified sterilizing liquid, there has been used a method where a sterilizing liquid is gasified in a high temperature atmosphere having a heating element by spraying a sterilizing liquid in an atomized form using a spray nozzle, or by dropping a sterilizing liquid in a droplet form using a dropping nozzle. Although the method which brings a sterilizing liquid into contact with the heating element by spraying a sterilizing liquid exhibits high gasification efficiency, there exists a possibility that the spray nozzle is clogged by a foreign material or by the precipitation of a stabilizing agent contained in a sterilizing liquid. To obviate these drawbacks, a nozzle (pipe) where an inner diameter of a nozzle distal end is set to 2 mm is used. The present inventors have developed a device where a tee's pipe is assembled into a middle portion of the nozzle, wherein compressed air for atomization is supplied from one inlet of the tee's pipe, hydrogen peroxide is supplied under pressure from the other inlet of the tee's pipe by a plunger pump, air and hydrogen peroxide are mixed together, the mixture of air and hydrogen peroxide is sprayed in a mist form from a distal end of the nozzle, and the mist is gasified by hot air. However, when compressed air and hydrogen peroxide are mixed together in this manner, a load which is at least equal to or more than a pressure of air is applied to the hydrogen peroxide supply pump. For example, to form a mist which can be instantaneously evaporated using a distal end of 2 mm, it is necessary to supply air at 50 L/min or more and air pressure at this point of time is approximately 0.1 MPa. Hydrogen peroxide is a foamable liquid and hence, bubbles are generated and grow due to friction in the pipe or natural decomposition during the supply of the liquid. When bubbles flow into a head of a pump, the bubbles generate an air cushion (so-called gas locking phenomenon) thus giving rise to a state where the liquid is not supplied. The poor quantitative supply performance of the pump exactly goes side by side with the danger of generating the insufficient sterilization, and stops a filling machine based on detection of an abnormal operation thus lowering the use ratio of the sterilizing liquid gasifying device.

To overcome this drawback, the present inventors have developed and put into practice a sterilizing liquid gasifying device which is disclosed in patent document 3 (Japanese Unexamined Patent Application Publication No. 2007-20744). In this sterilizing liquid gasifying device, however, there exists a possibility that a sterilizing liquid which is atomized by the spray nozzle collide with the hot air duct and hence, the sterilizing liquid atomized in advance returns again to liquid droplets. That is, there exists a possibility that the gasification efficiency is lowered. Accordingly, there is no way but to install a heating element (plate heater) on a bottom surface of the gasifying device as an auxiliary means so as to enable the transportation of a sterilizing liquid in a completely vaporized state to a container. Further, to increase the gasified amount, a contact time with hot air is prolonged by elongating a length of the hot air duct. These provisions inevitably make the sterilizing liquid gasifying device large-sized and increase the electric energy consumption due to poor gasification efficiency.

It is an object of the present invention to provide a sterilizing liquid gasifying device which is excellent in sterilizing liquid gasifying efficiency, consumes a small amount of electric energy, is compact in shape, and enables easy maintenance.

### Means to Solve the Object

The present inventors have made studies aiming at saving of electric energy consumption of a conventional sterilizing liquid gasifying device provided with a plate heater. Based on their experience that an atomized sterilizing liquid returns again to a sterilizing liquid in a liquid droplet form when sprayed, it has been considered that a plate heater is indispensable in this type of conventional sterilizing liquid gasifying device. On the other hand, the present inventors attempted, as a part of their studies, to develop a gasifying device which does not require a plate heater. When they supposed that a plate heater which is arranged on a bottom surface of a gasifying tank of a sterilizing liquid gasifying device is removed, the present inventors came up with an idea of mounting a spray nozzle on a center portion of a bottom plate of a gasifying tank such that the spray nozzle penetrates the bottom plate in order to allow an atomized sterilizing liquid (hydrogen peroxide, for example) collide with hot air in an opposed manner. The inventors carried out an experiment by mounting a spray nozzle on a center portion of a bottom plate of a gasifying tank such that the spray nozzle penetrates the bottom plate, and adjusting a spray opening portion on a distal end of the spray nozzle to be positioned in the inside of a hot air duct, while it was found that a sterilizing liquid atomized by the spray nozzle also collides with the hot air duct and the sterilizing liquid atomized in advance returns again to a liquid droplet state. When the present inventors were almost ready to give up on this idea relating to gasification of a collision direction spray type, by a chance, they found out that when the spray opening portion formed on the distal end of the spray nozzle is positioned below a hot air outlet which forms an opening portion of the hot air duct, and a sterilizing liquid is sprayed in the direction opposite to the flow of hot air ejected from a hot air opening so as to make an atomized sterilizing liquid generated by spraying collide with hot air, a completely gasified sterilizing liquid can be guided to a gas discharge opening, while a duct wall of the hot air duct at the hot air opening is not wetted with a sterilizing liquid. Based on such finding, the present invention has been completed.

That is, the present invention is directed to (1) a sterilizing liquid gasifying device comprising: a hot air duct having a hot air inlet at one end and a hot air outlet at the other end; a hot air source which supplies hot air having a temperature capable of gasifying a sterilizing liquid to the hot air inlet; a spray nozzle which sprays a sterilizing liquid in the direction opposite to the flow of hot air ejected from the hot air outlet so as to make a sprayed and atomized sterilizing liquid collide with hot air; and a hermetically sealed gasifying tank which surrounds at least the hot air outlet of the hot air duct and a spray portion at a distal end of the spray nozzle, wherein a gas discharge opening is formed on the gasifying tank on a side opposite to the spray portion at a distal end of the spray nozzle across the hot air outlet; and to (2) the sterilizing liquid gasifying device according to (1) wherein the spray nozzle is a spray nozzle having a gas-liquid mixture double pipe structure.

### Effect of the Invention

When a plate heater is not used in a conventional sterilizing liquid gasifying device of this type, it is inevitably necessary to set a temperature of hot air high for performing a large amount of gasification and hence, a temperature of a gas also becomes high eventually. Nevertheless, according to the sterilizing liquid gasifying device of the present invention, sterilizing-liquid gasification efficiency can be enhanced and hence, a large amount of gasification can be performed and thereby the number of gasifying units mounted on a filling machine can be decreased thus realizing a saving of space. Further, a predetermined gasification of sterilizing liquid can be performed even when a temperature of hot air is low and hence, a low-temperature gas can be also produced. Further, a plate heater which is indispensable in a conventional sterilizing liquid gasifying device of this type is no longer necessary and hence, the gasifying device becomes compact in shape, and the gasifying device can be automatically cleaned (automatic cleaning being not possible with a plate heater because of the waterproof property) thus realizing an ecological gasifying device (requiring no energy for heater) at a low price.

### Brief Description of Drawings

[Fig. 1]
   It is a longitudinal cross-sectional view of the sterilizing liquid gasifying device (collision direction spray type) of the present invention.
[Fig. 2]
   It is a system view showing a method of supplying a sterilizing liquid and air to the sterilizing liquid gasifying device (collision direction spray type) of the present invention.
[Fig. 3]
   It is an enlarged cross-sectional view of a spray nozzle having the gas-liquid mixture double pipe structure in the sterilizing liquid gasifying device (collision direction spray type) of the present invention.
[Fig. 4]
   It is a longitudinal cross-sectional view of a conventional sterilizing liquid gasifying device (horizontal direction spray type; comparison example 1 and comparison example 2/control).

### Mode of Carrying Out the Invention

The sterilizing liquid gasifying device of the present invention is not particularly limited provided that it is a sterilizing liquid gasifying device comprising: a hot air duct having a hot air inlet at one end and a hot air outlet at the other end; a hot air source which supplies hot air having a temperature capable of gasifying a sterilizing liquid to the hot air inlet; a spray nozzle which sprays a sterilizing liquid in the direction opposite to the flow of hot air ejected from the hot air outlet so as to make a sprayed and atomized sterilizing liquid collide with hot air; and a hermetically sealed gasifying tank which surrounds at least the hot air outlet of the hot air duct and a spray portion at a distal end of the spray nozzle, wherein a gas discharge opening is formed on the gasifying tank on a side opposite to the spray portion at a distal end of the spray nozzle across the hot air outlet, and a plate heater is not used. The above-mentioned spray nozzle is preferably a spray nozzle having a gas-liquid mixture double pipe structure, and particularly a spray nozzle having a gas-liquid mixture double pipe structure described in the above-mentioned patent document 3 (Fig. 3 of Japanese Unexamined Patent Application Publication No. 2007-20744).

In the sterilizing liquid gasifying device of the present invention, an amount of air supplied to the hot air duct can be set to 0.1 m³/min to 1 m³/min, and a pressure of compressed air supplied to the spray nozzle having a gas-liquid mixture double pipe structure can be set to 0.1 Mpa to 0.4 Mpa. Further, in the case where an amount of air to be supplied to the spray nozzle having the gas-liquid mixture double pipe structure is set to 1 m³/min and a pressure of compressed air supplied to the spray nozzle having the gas-liquid mixture double pipe structure is set to 0. 3 Mpa, a supply amount of a sterilizing liquid such as hydrogen peroxide (H₂O₂) is preferably set to 50 ml to 100 ml and a hot air inlet temperature is preferably set to 240°C to 440°C.

Although a shape of the gasifying tank is preferably a vertically cylindrical shape, the gasifying tank can have a vertical two-stage stepped cylindrical shape. When the gasifying tank has a vertical two-stage stepped cylindrical shape, it is constituted of a short vertically cylindrical large-diameter lower tank and a vertical cylindrical small-diameter upper tank mounted upright on a top wall of the short vertically cylindrical large-diameter lower tank. The hot air duct is preferably arranged concentrically with the gasifying tank, and a diameter ratio between the gasifying tank and the hot air duct is preferably set to approximately 20:10 to 20:16, and particularly to approximately 20:12 to 20:15. With respect to a distance between the hot air outlet of the hot air duct and the distal end of the spray nozzle, when they are too closely spaced, there exists a possibility that a duct wall of the hot air duct at the hot air outlet will be wetted, while when they are too largely spaced, hot air ejected from the hot air outlet flows to the gas discharge opening. Accordingly, for example, when a diameter of the hot air duct is approximately 60 mm, the distance between the hot air outlet of the hot air duct and the distal end of the spray nozzle is preferably set to 50 mm to 150 mm, and particularly to 90 mm to 110 mm.

Hereinafter, the present invention is explained in further detail in conjunction with the figures. Fig. 1 is a longitudinal cross-sectional view of a sterilizing liquid gasifying device (collision direction spray type) of the present invention. Fig. 2 is a system view showing a method of supplying a sterilizing liquid and air to the sterilizing liquid gasifying device (collision direction spray type) of the present invention. Fig. 3 is an enlarged cross-sectional view of a spray nozzle having the gas/liquid mixture double pipe structure in the sterilizing liquid gasifying device (collision direction spray type) of the present invention.

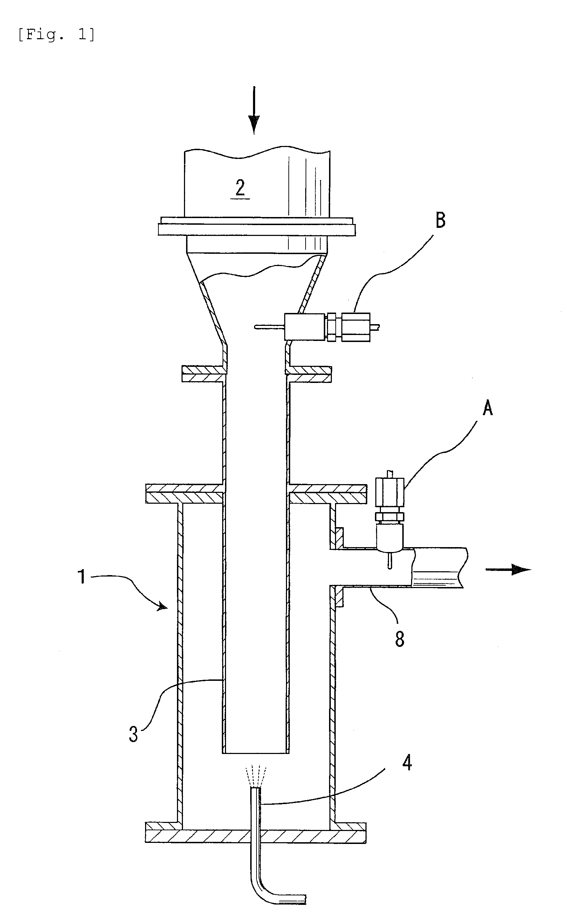

As shown in Fig. 1, the sterilizing liquid gasifying device comprises: a vertically cylindrical gasifying tank 1; a hot air duct 3 which supplies hot air generated by a hot air source 2 to the inside of the gasifying tank 1; and a spray nozzle 4 having a gas-liquid mixture double pipe structure which sprays a sterilizing liquid toward the hot air duct 3. The spray nozzle 4 comprises a vertically cylindrical nozzle body which penetrates a center portion of a bottom plate of the gasifying tank 1, and a spray portion at a distal end of the nozzle body. The hot air duct 3 which is connected to a lower portion of the hot air source 2 in a communicable state penetrates a top plate of the gasifying tank 1 and is suspended into the inside of the gasifying tank 1, which lower end (hot air outlet) reaches the vicinity of the spray portion at a distal end of the spray nozzle 4, and an outlet is opened. A discharge opening 8 for a sterilizing gas is formed on an upper end of a peripheral wall of the gasifying tank 1. An inlet end of a guide pipe is connected to the discharge opening 8, and an outlet end of the guide pipe is connected to a gas nozzle arranged above a container (not shown). Symbol A indicates a temperature sensor which measures a temperature of a sterilizing liquid in a gaseous form discharged through the discharge opening 8 (gas temperature), and symbol B indicates a temperature sensor which measures a temperature of hot air supplied to the hot air duct 3 from the hot air source 2 (heater temperature).

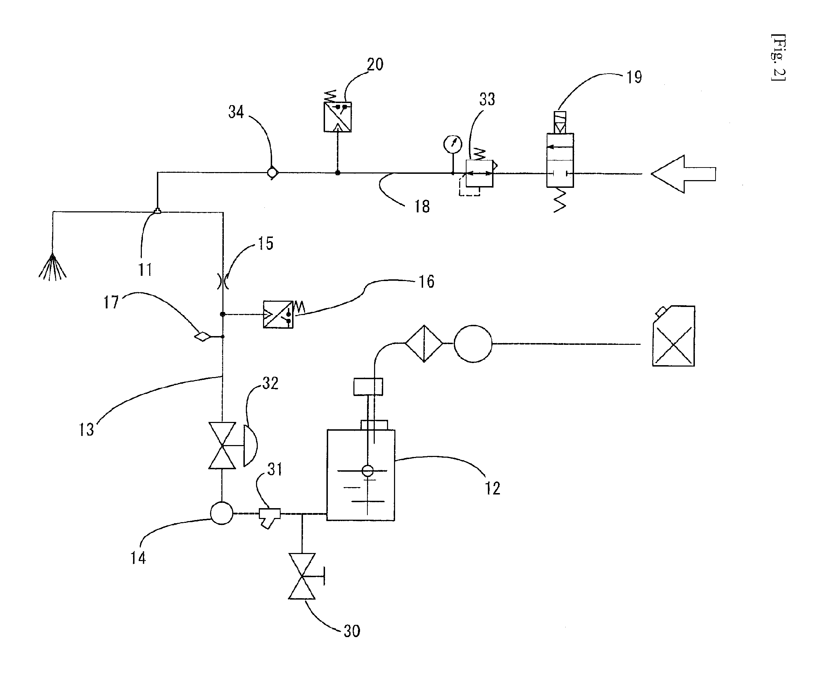

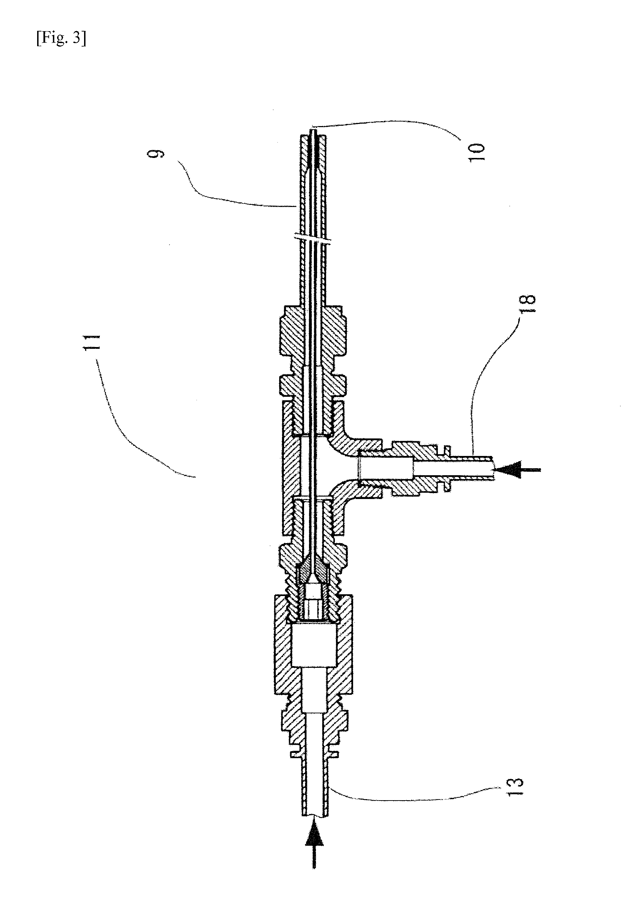

As shown in Fig. 2, the spray means according to one embodiment of the present invention comprises: a sterilizing agent tank 12 in which a sterilizing agent in a liquid form is stored; a spray nozzle having a gas-liquid mixture double pipe structure which sprays a sterilizing agent transported from the sterilizing agent tank 12 into the hot air duct; a guide pipe 13 which makes the sterilizing agent tank 12 and the spray nozzle communicate with each other; a plunger pump (transport means) 14 provided to an upstream portion of the guide pipe 13; a throttle means 15 provided to the guide pipe 13 on a downstream side of the plunger pump 14; a pressure sensor 16 provided in the vicinity of an upstream side of the throttle means 15; and an air chamber 17 provided to the guide pipe 13 between the plunger pump 14 and the throttle means 15. The sterilizing agent tank 12 is made of chemical-resistant stainless steel, for example, and can store a predetermined amount of sterilizing agent therein. The sterilizing agent in the sterilizing agent tank 12 is liquid level-controlled by a level sensor in such a manner that when an amount of sterilizing agent in the sterilizing agent tank 12 is decreased, the level sensor detects the decrease, and a sterilizing agent is supplied to the inside of the sterilizing agent tank 12 from a sterilizing agent source through a strainer which filters foreign materials. The guide pipe 13 connected to the sterilizing agent tank 12 is a resin-made or stainless-steel-made pipe having chemical resistance, for example, provided with a drain valve 30, a strainer 31, a plunger pump 14, a diaphragm valve 32, an air chamber 17, a pressure sensor 16 and the throttle means 15 from an upstream side (sterilizing agent tank 12 side). As shown in Fig. 3, the other end of the guide pipe 13 is connected to an inner pipe 10 of the spray nozzle having a gas-liquid mixture double pipe structure in a communicable state at a joint portion 11. In such case, a portion of the guide pipe 13 ranging from the plunger pump 14 to the throttle means 15 is installed such that a downstream side thereof is not oriented downwardly but is oriented at least horizontally or upwardly to prevent the stagnation of bubbles. As shown in Fig. 3, one end of an air supply pipe 18 on a downstream side is connected to an outer pipe 9 of the spray nozzle having the gas-liquid mixture double pipe structure in a communicable state at the joint portion 11, and the other end on an upstream side is connected to the pressurized air source not shown in the drawing. An electromagnetic valve 19, a pressure reducing valve 33, a pressure sensor 20 and a check valve 34 are provided sequentially from an air source side. Compressed air for atomizing a sterilizing agent supplied from an air source not shown in the drawing is controlled by the pressure reducing valve, passes through the check valve, passes through the outer pipe 9 of the spray nozzle and, thereafter, is mixed with a sterilizing agent transported in the guide pipe 13 and passed through the inner pipe 10 of the spray nozzle in the hot air duct to be formed into a mist by atomization.

When hot air is supplied to the hot air duct 3 from the hot air source 2 and, simultaneously, a mist of sterilizing liquid is sprayed from the spray nozzle 4 in the direction opposite to the flow of hot air ejected from the hot air outlet, the sprayed and atomized sterilizing liquid in a mist form collides with hot air ejected from the hot air outlet of the hot air duct 3 so that the sterilizing liquid is substantially completely gasified efficiently. The substantially completely gasified sterilizing liquid rises in a dispersed manner and is discharged from the gasifying tank 1 through the gas discharge opening 8. The sterilizing liquid in a gaseous form discharged from the discharge opening 8 is guided to a container by the guide pipe and is injected into the inside of the container by a gas nozzle.

Hereinafter, the performance of the sterilizing liquid gasifying device of the present invention is more specifically explained in accordance with examples. In a sterilizing liquid gasifying performance test, three kinds of sterilizing liquid gasifying devices in total comprising the sterilizing liquid gasifying device of the present invention and two kinds of conventional sterilizing liquid gasifying devices were used for the test.

### [Sterilizing liquid gasifying device (present invention)]

As the sterilizing liquid gasifying device, a device having the constitution shown in Fig. 1 to Fig. 3 was used, which is provided with a spray nozzle having a gas-liquid mixture double pipe structure for atomization (external mixing/outside air type: inner diameter of inner pipe: 2 mm, inner diameter of outer pipe: 3 mm, inner pipe projecting longer than outer pipe by 2 mm) is mounted, wherein a distance between the lower end (hot air outlet) of the hot air duct and the distal end (spray opening) of the spray nozzle is set to 100 mm, and a capacity of the gasifying tank is 1500 cm³.

### [Sterilizing liquid gasifying device (comparison example 1)]

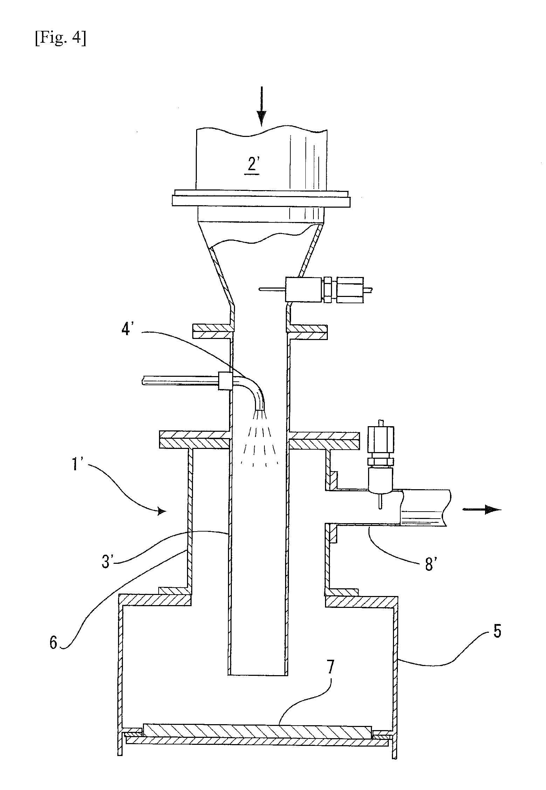

As the sterilizing liquid gasifying device of the comparison example 1, a device (horizontal direction spray type) shown in Fig. 4 was used, which is provided with a spray nozzle having a gas-liquid mixture double pipe structure for atomization shown in Fig. 3 (external mixing/outside air type: inner diameter of inner pipe : 2 mm, inner diameter of outer pipe: 3 mm, inner pipe projecting longer than outer pipe by 3 mm). The sterilizing liquid gasifying device shown in Fig. 4 comprises: a gasifying tank 1', a hot air duct 3' which supplies hot air generated by a hot air source 2' to the inside of the gasifying tank 1'; and a spray nozzle 4' which sprays a sterilizing agent to the inside of the hot air duct 3'. The gasifying tank 1' is constituted of a short vertically cylindrical large-diameter lower tank 5 and a vertically cylindrical small-diameter upper tank 6 mounted upright on a top wall of the short vertically cylindrical large-diameter lower tank 5, and a plate heater 7 is mounted on a bottom surface of the lower tank 5. A discharge opening 8' for a sterilizing gas is formed on an upper end of a peripheral wall of the upper tank 6. The hot air duct 3' connected to a lower portion of the hot air source 2' in a communicable manner penetrates a top plate of the upper tank 6 and is suspended into the inside of the upper tank 6, which lower end reaches the vicinity of the plate heater 7 of the lower tank 5, and an outlet is opened. The device was used with an electric power source of the plate heater 7 held in an OFF state.

### [Sterilizing liquid gasifying device (comparison example 2/control)]

As the sterilizing liquid gasifying device of the comparison example 2, a device (horizontal direction spray type) shown in Fig. 4 was used, which is provided with a spray nozzle having an injection needle (internal mixing type where air and sterilizing liquid are mixed together in advance: injection needle having inner diameter of 0.3 mm and length of 30 mm; see patent document 4). The comparison example 2 is a case where the device is used with an electric power source of the plate heater held in an OFF state, and the control is a case where the device is used with a temperature of the plate heater set at 300°C.

### [Gasification performance test]

Hydrogen peroxide water containing 35 mass % of hydrogen peroxide was used as a sterilizing liquid. The sterilizing liquid is indicated as "H₂O₂ amount" in the following Tables 1 to 3 and was used within a range from 40 ml/min to 100 ml/min, and "compressed air pressure" which corresponds to a spraying speed of a sterilizing liquid was set to 0.3 MPa (0.2 MPa also being used in addition to 0.2 MPa only with respect to control) . An amount of air supplied to the hot air duct was set to 1 m³/min, and "heater temperature" at the inlet of the hot air duct was controlled within a temperature range indicated in the following Tables 1 to 3. "Gas temperature" at the gas discharge opening was measured, and the measured temperatures are shown in the following Tables 1 to 3. With respect to "○" and "×" in the "determination" of the present invention and the comparison examples, "o" means a state where a duct wall of the hot air duct at the hot air outlet is not wetted, and "×" means a state where the duct wall of the hot air duct at the hot air outlet is wetted so that the dropping of droplets to bottom of the gasifying tank occurs.

The results of the sterilizing liquid gasification performance test using the sterilizing liquid gasifying device (present invention) is shown in Table 1. The results of the sterilizing liquid gasification performance test using the sterilizing liquid gasifying device (comparison example 1) is shown in Table 2. The results of the sterilizing liquid gasification performance test using the sterilizing liquid gasifying device (comparison example 2/control) is shown in Table 3.

**Table 1**

| plate heater | H₂O₂ amount | compressed air pressure | gas temperature | heater temperature | determination |
|---|---|---|---|---|---|
| - | 50 ml/min | 0.3 MPa | 120°C | 245°C | ○ |
| | | | 130°C | 260°C | ○ |
| | | | 140°C | 268°C | ○ |
| | | | 150°C | 280°C | ○ |
| | | | 160°C | 290°C | ○ |
| | | | 170°C | 316°C | ○ |
| - | 60 ml/min | 0.3 MPa | 140°C | 291°C | ○ |
| | | | 150°C | 306°C | ○ |
| | | | 160°C | 317°C | ○ |
| - | 70 ml/min | 0.3 MPa | 160°C | 333°C | ○ |
| | | | 170°C | 353°C | ○ |
| | | | 180°C | 365°C | ○ |
| - | 80 ml/min | 0.3 MPa | 170°C | 375°C | ○ |
| | | | 180°C | 382°C | ○ |
| | | | 190°C | 398°C | ○ |
| | | | 200°C | 414°C | ○ |
| - | 90 ml/min | 0.3 MPa | 170°C | 403°C | ○ |
| | | | 180°C | 412°C | ○ |
| - | 100 ml/min | 0.3 MPa | 180°C | 438°C | ○ |

**Table 2**

| plate heater | H₂O₂ amount | compressed air pressure | gas temperature | heater temperature | determination |
|---|---|---|---|---|---|
| - | 40ml/min | 0.3MPa | 190°C | 272°C | ○ |
| | 45ml/min | 0.3MPa | 180°C | 280°C | ○ |
| | 45ml/min | 0.3MPa | 190°C | 295°C | ○ |
| - | 50ml/min | 0.3MPa | 180°C | 290°C | × |
| | | | 190°C | 303°C | × |
| | | | 200°C | 311°C | ○ |
| - | 55ml/min | 0.3MPa | 220°C | 347°C | ○ |
| - | 60ml/min | 0.3MPa | 205°C | 330°C | × |
| | | | 210°C | 340°C | × |
| | | | 220°C | 354°C | × |
| - | 65ml/min | 0.3MPa | 230°C | 390°C | × |
| | | | 235°C | 400°C | × |

**Table 3**

| plate heater | H₂O₂ amount | compressed air pressure | gas temperature | heater temperature | determination |
|---|---|---|---|---|---|
| - | 50 ml/min | 0.3 MPa | 150°C | 267°C | × |
| | | | 160°C | 282°C | × |
| | | | 170°C | 301°C | ○ |
| | | | 180°C | 312°C | ○ |
| - | 60 ml/min | 0.3 MPa | 170°C | 314°C | × |
| | | | 180°C | 330°C | × |
| | | | 190°C | 350°C | ○ |
| | | | 200°C | 367°C | ○ |
| - | 70 ml/min | 0.3 MPa | 170°C | 320°C | × |
| | | | 180°C | 342°C | × |
| | | | 190°C | 362°C | × |
| | | | 200°C | 381°C | ○ |
| | | | 210°C | 398°C | ○ |
| - | 80 ml/min | 0.3 MPa | 190°C | 373°C | × |
| | | | 200°C | 391°C | × |
| | | | 210°C | 407°C | × |
| | | | 220°C | 422°C | ○ |
| 300°C | 50 ml/min | 0.3 MPa | 150°C | 228°C | ○ |
| | | 0.2 MPa | 150°C | 224°C | ○ |
| | 60 ml/min | 0.3 MPa | 150°C | 244°C | ○ |
| | | 0.2 MPa | 150°C | 241°C | ○ |
| | 70 ml/min | 0.3 MPa | 150°C | 265°C | ○ |
| | | 0.2 MPa | 150°C | 250°C | ○ |
| | 80 ml/min | 0.3 MPa | 150°C | 284°C | ○ |
| | | 0.2 MPa | 150°C | 265°C | ○ |
| | 90 ml/min | 0.3 MPa | 150°C | 305°C | ○ |
| | | 0.2 MPa | 150°C | 292°C | ○ |
| | | 0.3 MPa | 160°C | 328°C | ○ |
| | | 0.2 MPa | 160°C | 325°C | ○ |
| | 100 ml/min | 0.3 MPa | 160°C | 338°C | ○ |
| | | | 170°C | 358°C | ○ |
| | | | 180°C | 377°C | ○ |

From these results, it is understood that, with the use of the sterilizing liquid gasifying device of the present invention, although a plate heater is not provided, a gasification efficiency substantially equal to that of a conventional sterilizing liquid gasifying device provided with a plate heater (control) can be acquired, and a low temperature gas can be obtained when the "H₂O₂ amount" is relatively small, that is, 50 ml/min to 60 ml/min. Further, it is understood that, with the use of the sterilizing liquid gasifying device of the present invention, compared to a conventional sterilizing liquid gasifying devices which are not provided with the plate heater (comparison examples 1 and 2), the gasification efficiency is superior and, particularly, a low temperature gas can be produced, and further, even when the "H₂O₂ amount" is relatively large, a complete gasification can be attained.

### Industrial Applicability

The sterilizing liquid gasifying device of the present invention is useful in a field of machinery for filling a liquid such as milk or juice.

### Explanation of Letters or Numerals

1: gasifying tank
2: hot air source
3: hot air duct
4: spray nozzle
8: gas discharge opening
A: temperature sensor for measuring temperature of sterilizing liquid in a gaseous form (gas temperature)
B: temperature sensor for measuring temperature of hot air (heater temperature)

## Claims

1. A sterilizing liquid gasifying device comprising:
a hot air duct having a hot air inlet at one end and a hot air outlet at the other end;
a hot air source which supplies hot air having a temperature capable of gasifying a sterilizing liquid to the hot air inlet;
a spray nozzle which sprays a sterilizing liquid in the direction opposite to the flow of hot air ejected from the hot air outlet so as to make a sprayed and atomized sterilizing liquid collide with hot air; and
a hermetically sealed gasifying tank which surrounds at least the hot air outlet of the hot air duct and a spray portion at a distal end of the spray nozzle,
wherein a gas discharge opening is formed on the gasifying tank on a side opposite to the spray portion at a distal end of the spray nozzle across the hot air outlet.

2. The sterilizing liquid gasifying device according to claim 1, wherein the spray nozzle is a spray nozzle having a gas-liquid mixture double pipe structure.
